# EUROPEAN PATENT SPECIFICATION

(11) **EP 4 172 231 B1**
(45) Date of publication and mention of the grant of the patent: **22.05.2024**
(21) Application number: 21734130.4
(22) Date of filing: 21.06.2021
(51) Int. Cl.: C08G 18/64, C08G 79/025, C08G 18/79, C08G 65/333

(54) **POLYURETHANES BASED ON TERMINAL DIOL- AND DIAMINOPOLYPHOSPHAZENES AND THEIR HYDROGELS**
POLYURETHANE AUF DER BASIS VON TERMINALEN DIOL- UND DIAMINOPOLYPHOSPHAZENEN UND DEREN HYDROGELEN
POLYURÉTHANES À BASE DE DIOL ET DE POLYPHOSPHAZÈNES DE DIAMINE TERMINAUX ET LEURS HYDROGELS

(30) Priority: 26.06.2020 EP 20182531
(43) Date of publication of application: 03.05.2023
(73) Proprietor: Covestro Deutschland AG, 51373 Leverkusen (DE)
(72) Inventor: SUETTERLIN, Jan, 51061 Köln (DE); HECKROTH, Heike, 51519 Odenthal (DE); RICHTER, Frank, 51373 Leverkusen (DE); TEASDALE, Ian, 4040 Linz (AT); BRUEGGEMANN, Oliver, 4040 Linz (AT); PLAVCAN, Oliver, 4040 Linz (AT)
(74) Representative: Levpat
(86) International application number: PCT/EP2021/066799
(87) International publication number: WO 2021/259847

(56) References cited:
- CN-A- 103 804 628
- US-A1- 2017 183 453
- HARRY R. ALLCOCK ET AL: "Synthesis of Telechelic Polyphosphazenes via the Ambient Temperature Living Cationic Polymerization of Amino Phosphoranimines", MACROMOLECULES, vol. 32, no. 18, 1 September 1999 (1999-09-01), pages 5736-5743, XP055743267, WASHINGTON, DC, UNITED STATES ISSN: 0024-9297, DOI: 10.1021/ma990318f

## Description

The present invention relates to polyphosphazenes, polyurethanes based on these polyphosphazenes, methods for their manufacture and hydrogels based on the polyurethanes. The invention further relates to the use of the hydrogels in medical, veterinary and agricultural applications.

Poly(organo)phosphazenes are a family of inorganic molecular hybrid polymers with very diverse properties. The article "Preparation of polyphosphazenes: a tutorial review" by S. Rothemund and I. Teasdale, Chem. Soc. Rev., 2016, 45, 5200 gives an overview over the field.

WO 2001/07505 A1 relates to compositions of polyphosphazene-containing polymers and methods of preparation thereof. The disclosed compositions encompass telechelic functionalized polyphosphazenes and a variety of block and graft polyphosphazene-polystyrene, polyphosphazene-polysiloxane, and polyphosphazene-ROMP of norbornene copolymers. Methods for the preparation of such compositions generally involve generation of a polydichlorophosphazene species, attachment of a function group to the resultant polyphosphazene compound, and coupling the functionalized polyphosphazene with a corresponding organic or inorganic polymers or polymerizing the functionalized polyphosphazene with corresponding organic molecules.

WO 2013/190260 A2 discloses substituted poly(phosphazene) compounds comprising a combination of units having one or more of the structures (i) to (iii) 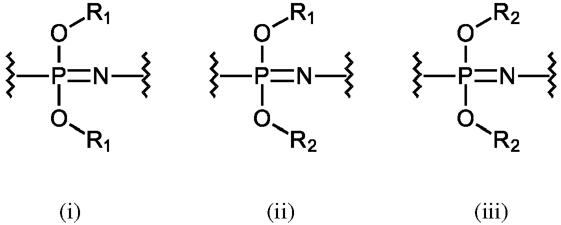 wherein: the combination comprises R₁ and R₂; each R₁, is independently an optionally substituted alkyl- or alkyl ether-based side chain containing an isocyanate-reactive moiety, an epoxide- reactive moiety, an amine-reactive moiety, a supramolecular noncovalent bonding moiety, or combinations thereof; and each R₂ is independently an optionally substituted alkyl- or alkyl ether- based side chain containing nitro, nitramine, nitrate ester, azide, an ammonium compound moiety with energetic counter-ion, or combinations thereof. Methods of making the compounds are also described.

WO 2015/192158 A1 relates to a polymer for tissue engineering from biodegradable polyphosphazenes, having photopolymerizable side groups, wherein the side groups of the polyphosphazenes are formed exclusively from amino acids and/or amino acid derivatives, which are bonded to the backbone of the polyphosphazene via the amino group of the amino acid and to a spacer attached to the acid group with a carbon chain of length m, which has a vinyl group at the free end, wherein m = 0 to m = 10.

Hydrogels are crosslinked 3-D polymeric networks with high water and biological liquids uptake capacity. They can structurally resemble soft tissue or a cell of the body and hydrogels have a wide application area such as drug delivery systems, tissue engineering, and wound dressings. Among these biomedical applications, polyurethane-based hydrogels have been widely used in drug release applications owing to their good mechanical strength and easy applicability to drugs and they are suitable for drug encapsulation, transportation and release.

Despite their formal similarity to peptide bonds, the urethane groups in polyurethanes have been found challenging to biologically degrade. A polyurethane hydrogel with increased susceptibility to hydrolysis would therefore be desirable.

Accordingly, a polyphosphazene as described in claim 1 is provided. This polyphosphazene bears NCO-reactive hydrogen atoms and can be cross-linked with polyisocyanates; this is the subject of claim 8 and method claim 9. The method according to claim 7 concerns the synthesis of polyphosphazenes with NCO-reactive hydrogen atoms. Hydrogels obtained from such polyurethanes are the subject of claim 12. Their uses are covered in claims 14 and 15. Advantageous embodiments are the subject of the dependent claims. They may be combined freely unless the context clearly indicates otherwise.

A polyphosphazene according to the invention has the general formula (I): 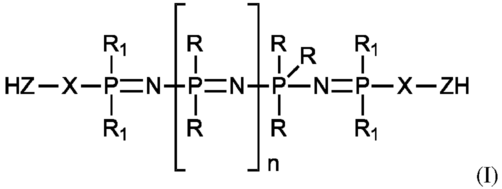 where each radical R is, independent of other radicals R, an organic radical which does not contain a terminal group with an NCO-reactive hydrogen atom; each radical R₁ is, independent of other radicals R₁, an organic rest; each radical X is, independent of other radicals X, an organic rest and n is zero or a positive integer and furthermore has the property that each radical ZH is, independent of other radicals ZH, a group comprising an NCO-reactive hydrogen atom.

Without wishing to be bound by theory it is believed that the polyphosphazenes according to the invention advantageously combines the reactivity with NCO groups to form polyurethanes and the possibility of hydrolytic cleavage to give a degradable or even bio-degradable polymer. Furthermore, such polyurethanes may be useful in the formation of hydrogels.

The degree of polymerization may be such that the number n in formula (I) is ≥ 3 to ≤ 1000 and preferably ≥ 10 to ≤ 100.

As used herein, the term "NCO-reactive hydrogen atom" denotes a hydrogen atom in the molecule which is able to react with an isocyanate functional group. The most common NCO-reactive hydrogen atoms are those bond to nitrogen, oxygen or sulfur atoms.

In the polyphosphazene according to the invention it is provided that the radicals R which are bond to the backbone of the polymer chain do not contain terminal groups with NCO-reactive hydrogen atoms. This is in contrast to the polymers of WO 2013/190260 A2 and leads to predominantly linear or fully linear polyurethanes.

A terminal group in the radical R is understood to be a functional group attached to the carbon atom within R having the most carbon-carbon bonds between itself and the phosphorus atom to which R is connected. In the event that the functional group is bound to a cyclic structure, positional isomers are disregarded. Hence, in an OH group bound to a phenyl group it would not make a difference whether the OH group is in ortho, meta or para-position to the remainder of R bound to the phenyl group.

The individual radicals R may be branched or unbranched and may comprise substituted or unsubstituted alkylene groups and/or substituted or unsubstituted arylene groups.

The individual radicals R may comprise a heteroatom such as N, O or S with which R is bonded to the respective phosphorus atom. This can be achieved by synthetic pathways where an amide, alkoxide or thiolate nucleophile substitutes a leaving group such as a chloride anion previously bond to the phosphorus atom.

Preferably the individual radicals R of the polyphosphazene (I) do not contain any NCO-reactive hydrogen atoms, in particular do not contain an OH group, an SH group or a primary amino group. It is noted that in a secondary amino group which can be formed by the reaction of a primary amino group with a chlorophosphazene group in the presence of a base (for example, with glycine methyl ester or 3-aminopropyl morpholine as the amine starting material) there is not considered to be an NCO-reactive hydrogen atom.

The individual radicals R₁ may be branched or unbranched and may comprise substituted or unsubstituted alkylene groups and/or substituted or unsubstituted arylene groups.

The individual radicals R₁ may comprise a heteroatom such as N, O or S with which R₁ is bonded to the respective phosphorus atom. This can be achieved by synthetic pathways where an amide, alkoxide or thiolate nucleophile substitutes a leaving group such as a chloride anion previously bond to the phosphorus atom.

The individual radicals R₁ may be the same as the individual radicals R or they may be different. It is preferred that the individual radicals R₁ are different from the individual radicals R.

The individual radicals R and R₁ may comprise hydrophilic moieties such as polyether groups or ionically charged groups.

The individual radicals X may be branched or unbranched and may comprise substituted or unsubstituted alkylene groups and/or substituted or unsubstituted arylene groups.

The individual radicals X may comprise a heteroatom. It is preferred that X is bonded to its respective phosphorus atom via a carbon atom.

In the individual radicals ZH the placeholder "Z" is meant to denote a heteroatom such as nitrogen, oxygen or sulfur to which the NCO-reactive hydrogen atom is bonded. "Z" is not meant to comprise further atoms which are part of the connection to the terminal phosphorus atom. These atoms would be grouped into "X".

In one embodiment at least one radical ZH is a hydroxyl group, a primary amino group or a secondary amino group. Preferred are the cases where all radicals ZH are hydroxyl groups or all radicals ZH are primary amino groups.

In another embodiment at least one radical X comprises one of the following groups: -B-CH₂-CH₂-, -Si-CH₂-CH₂- or -S-CH₂-CH₂-, wherein the -CH₂-CH₂- groups are located between the B, Si or S atoms and a P atom to which radicals R₁ are bond. This structural feature can be created by addition of a molecule with a B-H, Si-H or S-H bond to a C=C double bond. In the case of an S-H bond this may be designated as a thiol-ene addition. The P atom to which radicals R1 are bond is a terminal P atom of the polyphosphazene chain. Ultimately, the C=C double bond precursor group is bond to the polyphosphazene chain before addition of the B/Si/S-H group containing compound.

In another embodiment least one radical X comprises one of the following groups: -(CH₂)ₘ-B-, -(CH₂)ₘ-Si- or -(CH₂)ₘ-S- with m being an integer from 1 to 16, wherein the -(CH₂)ₘ- groups are located between the B, Si or S atoms and a radical ZH. This structural feature can be created starting from a compound with a HZ-(CH₂)ₘ-B/Si/S-H moiety which is then added to the polyphosphazene chain bearing a C=C double bond. Preferred values for m are ≥ 2 to ≤ 10. Particularly preferred are groups derived from mercaptoalkanols with 2, 3, 4, 5 or 6 carbon atoms or groups derived from mercaptoalkaneamines with 2, 3, 4, 5 or 6 carbon atoms.

In another embodiment at least one radical X-ZH comprises the group -Ar-CH₂-CH₂-S-(CH₂)ₘ-OH or -Ar-CH₂-CH₂-S-(CH₂)ₘ-NH₂ with m being an integer from 1 to 16 and Ar being a phenyl ring with or without additional substituents. This structural feature may be created by the reaction of a mercaptoalkanol or mercaptoalkaneamine with a styrene moiety bond to a terminal P atom of the polyphosphazene chain. Preferred values for m are ≥ 2 to ≤ 10. Particularly preferred are groups derived from mercaptoalkanols or mercaptoalkaneamines with 2, 3, 4, 5 or 6 carbon atoms.

In another embodiment at least one radical R comprises an oxygen or nitrogen atom bound to a phosphorus atom of a phosphazene group and/or at least one radical R₁ is a substituted or unsubstituted phenyl group. Unsubstituted phenyl groups for R₁ are preferred for reasons of commercial availability. With respect to R, a preferred radical group is -O-methyl/ethyl/propyl/butyl. Particularly preferred are ethoxy radicals for R. Another preferred group of radicals R derives from amino acid esters such as glycine esters, alanine esters, valine esters, isoleucine esters, leucine esters, methionine esters and phenylalanine esters. Examples for an alcohol moiety which is part of the amino acid esters include methyl, ethyl, propyl and butyl. If a radical R is said to derive from an amino acid ester, R would be the amino acid ester minus a hydrogen atom formerly bond to the amino group nitrogen atom of the amino acid. A third group of preferred radicals R are those derived from alkylaminomorpholines such as *N*-(3-aminopropyl) morpholine.

The present invention also concerns a method of manufacturing a polyphosphazene according to the general formula (II). The method comprises reacting a C=C double bond-carrying compound of the general formula (III) with an element-hydrogen bond-carrying compound of the general formula H-E-L-ZH: 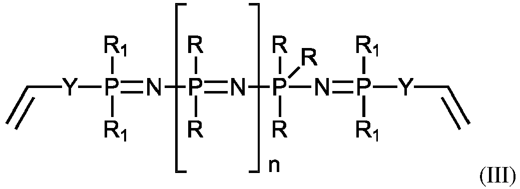 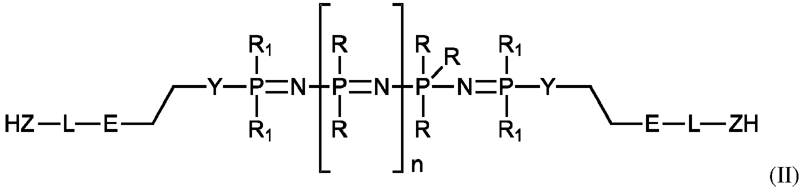 where: each radical E is, independent of other radicals E, B, Si or S; each radical L is, independent of other radicals L, an organic radical; each radical ZH is, independent of other radicals ZH, a group comprising an NCO-reactive hydrogen atom; each radical R is, independent of other radicals R, an organic radical which does not contain a terminal group with an NCO-reactive hydrogen atom; each radical R₁ is, independent of other radicals R₁, an organic rest; each radical Y is, independent of other radicals Y, an organic rest and n is zero or a positive integer.

The target molecule (II) may be seen as a case of a polyphosphazene of formula (I) where the radical X is the group Y-CH₂-CH₂-E-L. The reaction of H-E-L-ZH with (III) may be described, depending on the nature of E, a hydroboration, hydrosilylation or thiol-ene addition. Particularly preferred are mercaptoalkanols or mercaptoalkaneamines with 2, 3, 4, 5 or 6 carbon atoms as H-E-L-ZH.

The degree of polymerization may be such that the number n in formulae (II) and (III) is ≥ 3 to ≤ 1000 and preferably ≥ 10 to ≤ 100. The definitions and preferred embodiments for R and R₁ correspond to those outlined in conjunction with formula (I) above and shall not be repeated in the interest of brevity.

The radical Y is preferably a substituted or unsubstituted phenyl group. This has the advantage of the commercial availability of styrenic synthesis building blocks.

The reaction from (II) to (III) may take place under irradiation with UV light and/or in the presence of radical starters or catalysts if desired.

A polyurethane polymer can be obtained by reacting a polyphosphazene (I) according to the invention with a polyisocyanate. Preferably the polyphosphazene is of the structure (II).

Examples for suitable polyisocyanates include methylene diphenyl diisocyanate (MDI), methylene dicyclohexyl diisocyanate (H12-MDI), polymeric MDI, toluylene diisocyanate (TDI), xylylene diisocyanate (XDI), pentamethylene diisocyanate (PDI), hexamethylene diisocyanate (HDI), isophorone diisocyanate (IPDI) and a mixture comprising at least two of the aforementioned polyisocyanates. Further suitable polyisocyanates include the prepolymers, isocyanurates, iminooxadiazinediones, allophanates, biurets and uretdiones of the afore-mentioned polyisocyanates. The polyurethane polymer may include polyol-derived building blocks different from polyphosphazenes (I) such as polyether polyol or polyester polyol building blocks, if desired. The polymer may be unfoamed or foamed material.

A method of manufacturing a polyurethane polymer comprises reacting a polyphosphazene (I) according to the invention with a polyisocyanate. Preferably the polyphosphazene is of the structure (II). Reference is made to the polyisocyanates mentioned above. The reaction mixture comprising the polyphosphazene (I) and the polyisocyanate may furthermore include polyester polyols and/or polyether polyols. With respect to catalysts, tin-free catalysts are preferred, especially if the polyurethane is to be used in medical applications. The NCO index (molar ratio of NCO groups to NCO-reactive H-atoms) in the reaction mixture may, for example, be in a range of 90 to 110. If desired, chemical and/or physical blowing agents may be added to the reaction mixture. Mechanical frothing of the reaction mixture is also possible.

In the event that the polyphosphazene comprises hydrophilic moieties, an aqueous preparation of the polyphosphazene may be reacted with the polyisocyanate. This allows for the in situ formation of a hydrogel. Alternatively or additionally, an aqueous preparation of the polyisocyanate may be used. However, careful reaction timing would then be required owing to the reactivity of polyisocyanates with water.

In an embodiment of the polyurethane polymer or the method of its manufacture the polyisocyanate is an NCO-terminated prepolymer obtained by the reaction of a monomeric polyisocyanate and a polyether polyol. Polyether polyols are advantageous as their hydrophilicity facilitates the formation of hydrogels. Preferred are poly(oxyethylene) and poly(oxyethylene-co-propylene) polyols such as those obtained via double metal cyanide (DMC) catalysis. Particularly preferred are polyether polyols with ≥ 60 weight-% or ≥ 70 weight-% of ethylene oxide units, based on the total weight of the polyether polyol. Hydroxyl numbers (DIN 53240) of the polyol may be in the range of ≥ 20 mg KOH/g to ≤ 250 mg KOH/g, preferably ≥ 25 mg KOH/g to ≤ 50 mg KOH/g.

The polyol is preferably started on a short-chained polyalkylene oxide, for example on a glycerine-started poly(oxypropylene)triol with a hydroxyl number of ≥ 250 mg KOH/g to ≤ 400 mg KOH/g or a propylene glycol-started poly(oxypropylene)diol with a hydroxyl number of ≥ 200 mg KOH/g to ≤ 300 mg KOH/g.

With respect to the monomeric polyisocyanate, aliphatic diisocyanates such as pentamethylene diisocyanate, hexamethylene diisocyanate, isophorone diisocyanate, methylene dicyclohexyl diisocyanate and their isocyanurates, iminooxadiazinediones, allophanates, biurets and uretdiones are preferred. The NCO content of the prepolymer (DIN EN ISO 11909) may be in a range of ≥ 2% to ≤ 5%.

In another embodiment of the polyurethane polymer or the method of its manufacture the polyether polyol is a random copolymer of an alkylene oxide and a comonomer selected from: lactides, glycolides, cyclical dicarboxylic acid anhydrides and mixtures of at least two of the aforementioned compounds. These polyols have been found to be biodegradable owing to the incorporation of the comonomers. Details, including their synthesis, can be found in the published patent application WO 2013/092504 A1. It is preferred that the alkylene oxide is ethylene oxide and/or a mixture of ethylene oxide and propylene oxide. Hydroxyl numbers (DIN 53240) of the polyol may be in the range of ≥ 20 mg KOH/g to ≤ 250 mg KOH/g, preferably ≥ 25 mg KOH/g to ≤ 50 mg KOH/g.

The polyol is preferably started on a short-chained polyalkylene oxide, for example on a glycerine-started poly(oxypropylene)triol with a hydroxyl number of ≥ 250 mg KOH/g to ≤ 400 mg KOH/g or a propylene glycol-started poly(oxypropylene)diol with a hydroxyl number of ≥ 200 mg KOH/g to ≤ 300 mg KOH/g. The number average molecular weight of the polyol, as determined by gel permeation chromatography (THF as eluent, polystyrene standards), may be ≥ 200 g/mol to ≤ 10000 g/mol and preferably ≥ 1000 g/mol to ≤ 8000 g/mol.

The comonomer content may be ≥ 5 weight-% to ≤ 40 weight%, preferably ≥ 10 weight-% to ≤ 30 weight%, based on the total weight of the polyether polyol.. The comonomers may be used in their monomeric, dimeric, trimeric or polymeric forms, for example as dilactide in the case of lactides.

A hydrogel can be obtained by contacting a polyphosphazene (I) according to the invention and/or a polyurethane according to the invention with water. This is easily achievable by immersing the polyphosphazene or polyurethane in water. The water may be buffered, for example to a pH of 7.4.

In an embodiment the hydrogel further comprises, distributed within the hydrogel, a pharmaceutical compound, a pesticide, a herbicide, a pheromone or a combination of at least two of the aforementioned compounds.

Suitable pharmaceutical compounds include:
1. Anti-infectives, such as antibiotics, including penicillin, tetracycline, chlortetracycline bacitracin, nystatin, streptomycin, neomycin, polymyxin, gramicidin, oxytetracycline, chloramphenicol, and erythromycin; sulfonamides, including sulfacetamide, sulfamethazine, sulfadiazine, sulfamerazine, sulfamethizole and sulfisoxazole; antivirals, including idoxuridine; and other anti-infectives including nitrofurazone and sodium propionate.
2. Anti-allergenics such as antazoline, methapyrilene, chlorpheniramine, pyrilamine and prophenpyridamine.
3. Anti-inflammatories such as hydrocortisone, cortisone, dexamethasone 21-phosphate, fluocinolone, triamcinolone, medrysone, prednisolone, prednisolone 21-phosphate, and prednisolone acetate.
4. Decongestants such as phenylephrine, naphazoline, and tetrahydrazoline.
5. Miotics and antichlolinesterases such as pilocarpine, eserine salicylate, carbachol, diisopropyl fluorophosphate, phospholine iodide, demecarium bromide, physostigmin and neostigmin.
6. Mydriatics such as atropine sulfate, cyclopentolate, homatropine, scopolamine, tropicamide, eucatropine, and hydroxyamphetamine.
7. Sympathomimetics such as epinephrine.
8. Neuroleptics such as reserpine and chlorpromazine.
9. Estrogens such as estrone, 17*β*-estradiol, ethinyl estradiol, and diethyl stilbesterol.
10. Progestational agents such as progesterone, 19-norprogesterone, norethindrone, megestrol, melengestrol, chlormadinone, ethisterone, medroxyprogesterone, norethynodrel and 17*α-*hydroxy-progesterone.
11. Humoral agents such as the prostaglandins, for example, PGE1, PGE2, and PGF2.
12. Antipyretics such as acetylsalicylic acid, sodium salicylate, and salicylamide.
13. Antispasmodics such as atropine, methantheline, papaverine, and methscopolamine bromide.
14. Cardioactive agents such as benzydroflumethiazide, flumethiazide, chlorothiazide, and aminotrate.

One type of preferred pharmaceutical compounds is a progestogen or a drug having a progestogenic activity. In particular the compound may be selected from the group of progesterone and its derivatives, cyproterone acetate, desogestrel, etonogestrel, levonorgestrel, lynestrenol, medroxyprogesterone acetate, norethisterone, norethisterone acetate, norgestimate, drospirenone, gestodene, 19-nor-17-hydroxy progesterone esters, 17*α-*ethinyltestosterone and derivatives thereof, 17*α*-ethinyl-19-nor-testosterone and derivatives thereof, ethynodiol diacetate, dydrogesterone, norethynodrel, allylestrenol, medrogestone, norgestrienone, ethisterone, dl-norgestrel or a mixture of at least two of the aforementioned compounds.

Another type of preferred pharmaceutical compound is a drug capable of preventing or suppressing endometrial bleeding. In particular the compound may be selected from the group of prostaglandin synthesis inhibitors, NSAIDs, inhibitors of leukotriene, oxytocin antagonists, pancreatic trypsin inhibitors, COX-inhibitors, antifibrinolytic drugs, estrogens, antiestrogens, aromatase inhibitors, cytokine inhibitors, glucocorticoids, progestogens with pronounced glucocorticoid acticity, danazol, gestrinone, angiogenesis inhibitors or a mixture of at least two of the aforementioned compounds.

It is possible to combine the progestogen or a drug having a progestogenic activity with the drug capable of preventing or suppressing endometrial bleeding. Particularly preferred is the combination of levonorgestrel with tranexamic acid, mefenamic acid, danazol or an angiogenesis inhibitor. This is useful in an intrauterine device.

Another type of preferred pharmaceutical compound is a calcium channel blocker, in particular nimodipine or nifedipine.

Another type of preferred pharmaceutical compound is a cytostatic agent such as cisplatin, rituximab, bevacizumab, trastuzumab, imatinib, lenalidomide, pemetrexed, bortezomib, cetuximab, leuprolein or abiraterone.

Lastly, opioid analgetics and non-opioid analgetics are also a type of preferred pharmaceutical compound.

The pharmaceutical compounds (drugs) can be in different forms, such as uncharged molecules, components of molecular complexes, or non-irritating, pharmacologically acceptable salts such as hydrochloride, hydrobromide, sulphate, phosphate, nitrate, borate, acetate, maleate, tartrate, salicylate, etc. For acidic drugs, salts of metals, amines, or organic cations (e.g., quaternary ammonium) can be employed. Furthermore, simple derivatives of the drugs (such as ethers, esters, amides, etc.) which have desirable retention and release characteristics but which are easily hydrolyzed by body pH, enzymes, etc., can be employed.

Also contemplated are a hydrogel according to the invention for use as a post-operative separator between internal organs of a human or an animal and a hydrogel according to the invention for use as a delayed-release formulation for the pharmaceutical compound, pesticide, herbicide, pheromone or combination of at least two of the aforementioned compounds distributed within the hydrogel.

Post-operative separators (adhesion barriers) can be used to reduce or prevent unwanted tissue adhesions in a patient after surgery by separating the internal tissues and organs while they heal. The prevention of intraabdominal adhesions is expressly contemplated.

In its use as a delayed release formulation the hydrogel can be viewed as a drug delivery device or system. Drug delivery systems of the invention can take a wide variety of shapes and forms for administering the drugs at controlled rates to different areas of the body. Thus, the invention includes external and internal drug-delivery systems such as skin patches, sublingual or buccal tablets, peroral dosage forms, implantates for releasing a drug in the tissues of a living organism, pessaries, prosthesis, artificial glands, vaginal or rectal suppositories, cervical rings, troches, drug-dispensing intrauterine devices and ocular inserts.

### Examples

The present invention will be further described in the following examples and figures without wishing to be limited to them.

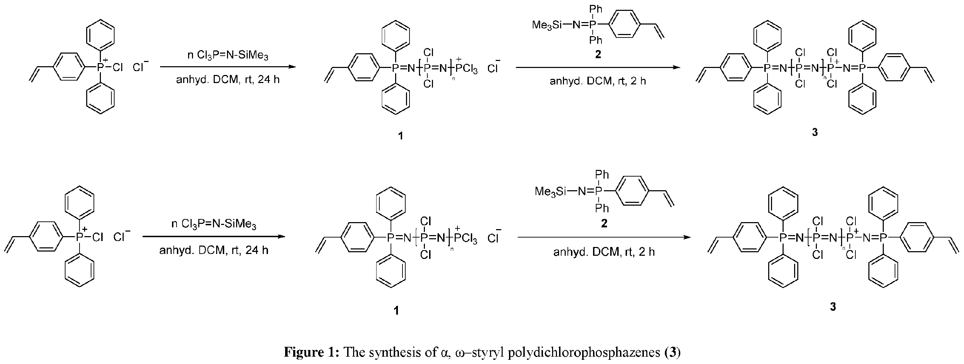

### Synthesis of α, ω-distyryl poly[(dichloro)phosphazenes] (3) - Figure 1

The synthesis of the chlorinated, styrene-functionalized λ⁵-phosphane mediator was carried out according to literature (Wilfert, S.; Henke, H.; Schoefberger, W.; Brüggemann, O.; Teasdale, I., Chain-End-Functionalized Polyphosphazenes via a One-Pot Phosphine-Mediated Living Polymerization. Macromol. Rapid Commun. 2014, 35 (12), 1135-1141).

4-(Diphenylphosphino)styrene (0.58 g, 2.01 mmol, 1 equiv.) and hexachloroethane (0.52 g, 2.21 mmol, 1.1 equiv.) were dissolved in 2 mL of anhydrous dichloromethane and stirred at room temperature for 24 h to yield dichlorodiphenyl(4-vinylphenyl)-λ⁵-phosphane, which was used for subsequent reactions without further purification. Yield: quantitative; ³¹P{¹H} NMR (121 MHz, CDCl₃, δ): 63.33 (R₃P=N-) ppm.

Dichlorodiphenyl(4-vinylphenyl)-λ⁵-phosphane (2.01 mmol, 1 equiv.) was reacted with N-(trimethylsilyl)-trichlorophosphoranimine (3.16 g, 14.08 mmol, 7 equiv.) in 5 mL of anhydrous dichloromethane and stirred at room temperature for 12 h to yield α-poly[(dichloro)phosphazene] **1**, which was used for subsequent reactions without further purification. Yield: quantitative; ³¹P{¹H} NMR (121 MHz, CDCl₃, δ): -18.19 (-Cl₂P=N-), 8.09 (-PCl₃), 19.50 (Ph₃P=N-) ppm.

4-(Diphenylphosphino)styrene (0.87 g, 3.08 mmol, 1 equiv.) and trimethylsilyl azide (1.39 g, 12.07 mmol, 4 equiv.) were dissolved in 25 mL of anhydrous dichloromethane. According to the ³¹P NMR spectrum, the reaction was completed after stirring for 96 h at room temperature. Next, vacuum distillation was used to remove the excess of the azide in the solution after the reaction. This was achieved by transferring the solution into a Schlenk Tube and evaporating the solvent under reduced pressure obtaining the yellow, waxy-solid 1,1-diphenyl-*N*-(trimethylsilyl)-1-(4-vinylphenyl)-λ⁵-phosphanimine (*N*-organophosphoranimine) **2**. Yield: 0.76 g (87 %); ¹H NMR (300 MHz, CDCl₃, δ): -0.18 (s, 1.5H, -CH₃), -0.05 (s, 6H, -CH₃), 0.08 (s, 0.5H, -CH₃), 0.17 (s, 1H, -CH₃), 5.34 (dd, 1H, -CH=*CH₂*), 5.83 (dd, 1H, -CH=*CH₂*), 6.74 (dd, 1H, -*CH*=CH₂), 7.58 (m, 14H, -Ph₃) ppm; ³¹P{¹H} NMR (121 MHz, CDCl₃, δ): 0.49 (R₃P=N-) ppm.

The *N*-organophosphoranimine **2** (1.13 g, 3.02 mmol, 1.5 equiv.) was dissolved in 8 mL of anhydrous dichloromethane, added to the solution of α-poly[(dichloro)phosphazene] **3** (2.01 mmol, 1 equiv.) in anhydrous dichloromethane and stirred at room temperature for 24 h until completion of the reaction, based on the ³¹P NMR spectrum. The obtained solution of the end-capped α-ω-distyryl poly[(dichloro)phosphazene] **3** was used for subsequent reactions without further purification. Yield: quantitative; ³¹P{¹H} NMR (121 MHz, CDCl₃, δ): -18.21 (-Cl₂P=N-), 18.01 (-N=PPh₃), 19.48 (Ph₃P=N-) ppm.

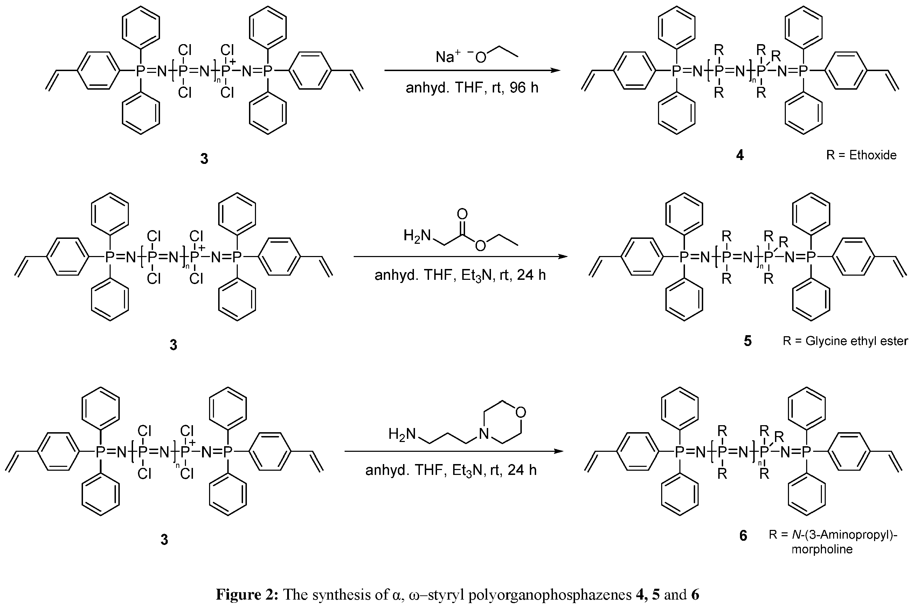

### Synthesis of α, ω-distyryl polyorganophosphazenes 4, 5 and 6 - Figure 2

Starting from **3**, α, **ω-distyryl** polyorganophosphazenes were synthesized as follows:
The synthesis of α-ω-distyryl poly[bis(ethoxy)phosphazene] **4** was carried as follows: Sodium ethoxide was generated *in situ* by gradually dissolving sodium metal (1.17 g, 50.81 mmol, 1 equiv.) in anhydrous ethanol (29.67 mL, 0.54 mol, 10 equiv.) and stirring the solution for 48 h under a nitrogen stream, which was used to evaporate unreacted amounts of ethanol after the sodium metal was completely dissolved in the solution. The obtained solid white, dry sodium ethoxide (3.46 g, 50.81 mmol, 24.5 equiv.) was dissolved in 50 mL of anhydrous tetrahydrofuran and a solution of α-ω-distyryl poly[(dichloro)phosphazene] (1 equiv.) in 10 mL of anhydrous dichloromethane was added dropwise. According to the ³¹P NMR spectrum, the reaction was completed after stirring for 96 h at room temperature. The solution was then centrifuged, the precipitate removed and the solvent was removed under reduced pressure to yield a solid-waxy brown-yellow product. Next, the polymer was dissolved in 10 mL of anhydrous ethanol and purified by dialysis against ethanol for 48 h (1 kDa cut-off). Finally, the solvent was removed under reduced pressure and the pale-yellowish product was dried further under vacuum at 45 °C for 24 h.

Yield: 1.40 g (41 %); ¹H NMR (300 MHz, CDCl₃, δ): 1.29 (br, 26H, -CH₂-*CH₃*), 4.15 (br, 18H, -*CH₂*-CH₃), 5.42 (br, 2H, -CH=*CH₂*), 5.88 (br, 2H, -CH=*CH₂*), 6.75 (br, 2H, -*CH*=CH₂), 7.61 (br, 28H, -Ph₃) ppm; ³¹P{¹H} NMR (121 MHz, CDCl₃, δ): -1.39 (-Cl₂P=N-), 13.76 (-N=PPh₃), 15.08 (Ph₃P=N-) ppm.

The synthesis of α-ω-distyryl poly[bis(glycine ethyl ester)phosphazene] **5** was synthesized as follows: Glycine ethyl ester hydrochloride (13.76 g, 98.57 mmol, 1 equiv.) was dissolved in 100 mL of anhydrous tetrahydrofuran, and triethylamine (27.25 mL, 0.197 mol, 2 equiv.) was added. The solution was stirred at room temperature for 48 h, filtered and the solvent removed under reduced pressure. The obtained glycine ethyl ester (5.24 g, 50.87 mmol, 24.5 equiv.) was dissolved in 40 mL of anhydrous tetrahydrofuran and triethylamine (14.05 mL, 0.102 mol, 49 equiv.) was added. Next, a solution of α-ω-distyryl poly[(dichloro)phosphazene] (1 equiv.) in 10 mL of anhydrous dichloromethane was added dropwise. According to the ³¹P NMR spectrum, the reaction was completed after stirring for 24 h at room temperature. The solution was then filtered and the solvent removed under reduced pressure to yield a waxy, yellow product. Next, the polymer was dissolved in 10 mL of anhydrous ethanol and purified by dialysis against ethanol for 48 h (1 kDa cut-off). Finally, the solvent was removed under reduced pressure and the orange-yellowish product was dried further under vacuum at 45 °C for 24 h.

Yield: 2.68 g (50 %); ¹H NMR (300 MHz, CDCl₃, δ): 1.22 (br, 60H, -CH₂-*CH₃*), 3.70 (br, 40H, -NH-*CH₂*-)*,* 4.11 (br, 45H, -*CH₂*-CH₃), 5.41 (br, 2H, -CH=*CH₂*), 5.88 (br, 2H, -CH=*CH₂*), 6.74 (br, 2H, -*CH*=CH₂), 7.60 (br, 28H, -Ph₃) ppm; ³¹P{¹H} NMR (121 MHz, CDCl₃, δ): 1.02 (-Cl₂P=N-), 10.78 (-N=PPh₃), 12.04 (Ph₃P=N-) ppm.

The α-ω-distyryl poly[bis(N-(3-aminopropyl)morpholine)phosphazene] **6** was prepared by dissolving N-(3-aminopropyl)morpholine (6.36 mL, 43.55 mmol, 24.5 equiv.) in 40 mL of anhydrous tetrahydrofuran and adding triethylamine (12.04 mL, 87.10 mol, 49 equiv.). Next, a solution of α-ω-distyryl poly[(dichloro)phosphazene] (1 equiv.) in 10 mL of anhydrous dichloromethane was added dropwise. According to the ³¹P NMR spectrum, the reaction was completed after stirring for 24 h at room temperature. The solution was then filtered and the solvent removed under reduced pressure to yield a waxy, orange-yellow product. Next, the polymer was dissolved in 10 mL of anhydrous ethanol and purified by dialysis against ethanol for 48 h (1 kDa cut-off). Finally, the solvent was removed under reduced pressure and the orange product was dried further under vacuum at 45 °C for 24 h.

Yield: 4.55 g (67 %); ¹H NMR (300 MHz, CDCl₃, δ): 1.67 (br, 26H, -CH₂-*CH₂*-CH₂-), 2.41 (br, 80H, *-CH₂-CH₂*NH-*CH₂*-)*,* 2.88 (br, 26H, -NH-*CH₂*-), 3.68 (br, 55H, *-CH₂*-O-*CH₂*-), 5.43 (br, 2H, -CH=*CH₂*), 5.88 (br, 2H, -CH=*CH₂*), 6.74 (br, 2H, -*CH*=CH₂), 7.59 (br, 28H, -Ph₃) ppm; ³¹P{¹H} NMR (121 MHz, CDCl₃, δ): -3.57 (-Cl₂P=N-), 11.25 (Ph₃P=N-) ppm.

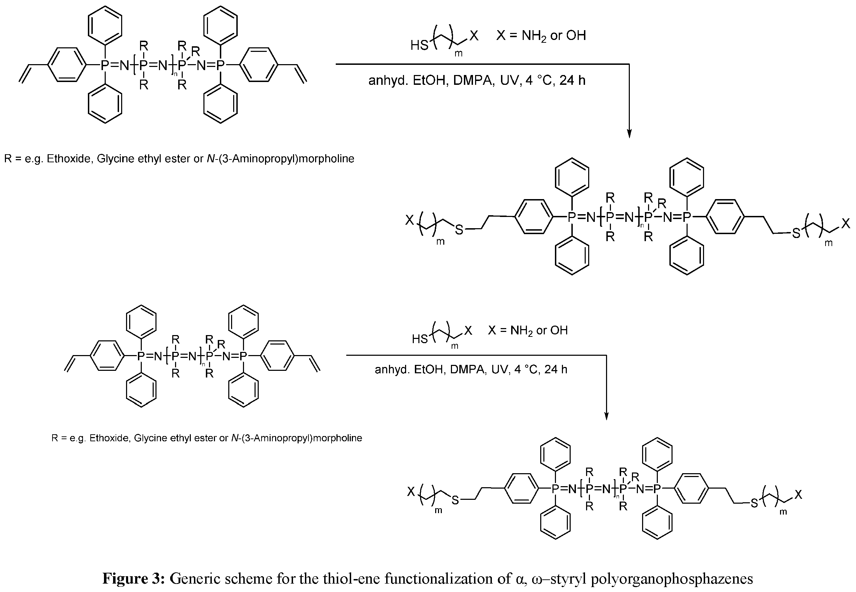

### Functionalization the α-ω-terminal double bonds of polymer 6 - Figure 3

The following procedure describes an example of functionalization the α-ω-terminal double bonds of polymer **6** using the thiol-ene photoreaction with 6-mercapto-1-hexanol.

α-ω-distyryl poly[bis(*N*-(3-aminopropyl)morpholine)phosphazene] (300 mg, 0.0885 mmol, 1 equiv.) and 2,2-dimethoxy-2-phenylacetophenone (30 g, 10 w%) were dissolved in 5 mL of anhydrous ethanol in a non-UV-absorbing flask. Next, 6-mercapto-1-hexanol (23.77 mg, 0.117 mmol, 2 equiv.) was added. The flask was then sealed with a rubber septum and flushed with argon for 10 minutes. The solution was then stirred and irradiated with UV light at 4 °C overnight. Completion of the reaction was controlled by ¹H NMR spectroscopy. After the reaction was finished, the solvent was removed under reduced pressure. 20 mL of dichloromethane were added to re-dissolve the obtained α-ω-diol poly[bis(*N*-(3-aminopropyl)morpholine)phosphazene] and the solvent was removed again under reduced pressure to remove ethanol residues in the polymer. Finally, the polymer was dissolved in anhydrous tetrahydrofuran and used for subsequent reactions without further purification. Yield: quantitative.

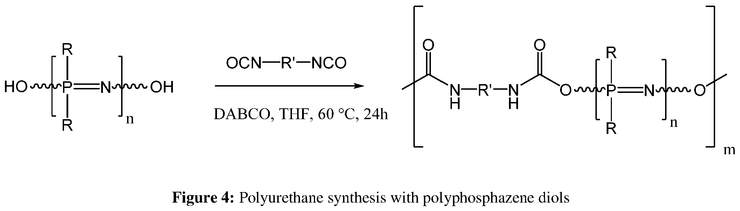

### Polyurethane synthesis with polyphosphazene diols - Figure 4

Poly(hexamethylene diisocyanate) (HDI biuret and oligomers; 1 NCO-equiv.) was added to a solution of α-ω-diol poly[bis(*N*-(3-aminopropyl)morpholine)phosphazene] (1 equiv.) in anhydrous tetrahydrofuran and stirred for 4 h at 50 °C. After the reaction, the solvent was removed under reduced pressure and the solid, orange-yellowish polyurethane was dried further under vacuum at 45 °C for 24 h. Yield: quantitative; FT-IR (*v*): 3273 (*-NH-*), 3057 (-*Ar-H*), 2932-2807 (-*CH₂*-), 1764 (-*C*=*O*-)*,* 1688 (-NH-CO-), 1525 (*-NH-*CO*-*), 1233 (*-P*=*N*-), 860 (*-P-N-*) cm⁻¹.

## Claims

1. A polyphosphazene of the general formula (I): 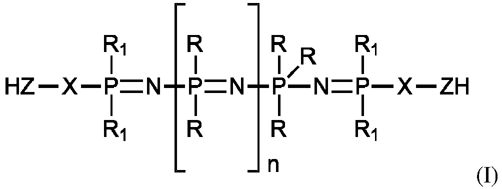 where:
each radical R is, independent of other radicals R, an organic radical which does not contain a terminal group with an NCO-reactive hydrogen atom;
each radical R₁ is, independent of other radicals R₁, an organic rest;
each radical X is, independent of other radicals X, an organic rest;
n is zero or a positive integer;
**characterized in that**
each radical ZH is, independent of other radicals ZH, a group comprising an NCO-reactive hydrogen atom.

2. The polyphosphazene according to claim 1, wherein at least one radical ZH is a hydroxyl group, a primary amino group or a secondary amino group.

3. The polyphosphazene according to claim 1 or 2, wherein at least one radical X comprises one of the following groups: -B-CH₂-CH₂-, -Si-CH₂-CH₂₋ or -S-CH₂-CH₂-, wherein the -CH₂-CH₂- groups are located between the B, Si or S atoms and a P atom to which radicals R₁ are bond.

4. The polyphosphazene according to one of claims 1 to 3, wherein at least one radical X comprises one of the following groups: -(CH₂)ₘ-B-, -(CH₂)ₘ-Si- or -(CH₂)ₘ-S- with m being an integer from 1 to 16, wherein the -(CH₂)ₘ- groups are located between the B, Si or S atoms and a radical ZH.

5. The polyphosphazene according to one of claims 1 to 4, wherein at least one radical X-ZH comprises the group -Ar-CH₂-CH₂-S-(CH₂)ₘ-OH or -Ar-CH₂-CH₂-S-(CH₂)ₘ-NH₂ with m being an integer from 1 to 16 and Ar being a phenyl ring with or without additional substituents.

6. The polyphosphazene according to one of claims 1 to 5, wherein at least one radical R comprises an oxygen or nitrogen atom bound to a phosphorus atom of a phosphazene group and/or at least one radical R₁ is a substituted or unsubstituted phenyl group.

7. A method of manufacturing a polyphosphazene according to the general formula (II), comprising reacting a C=C double bond-carrying compound of the general formula (III) with an element-hydrogen bond-carrying compound of the general formula H-E-L-ZH: 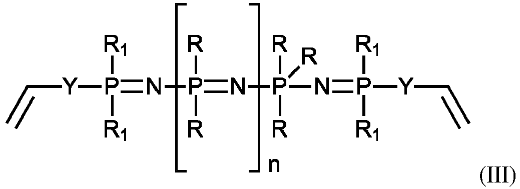 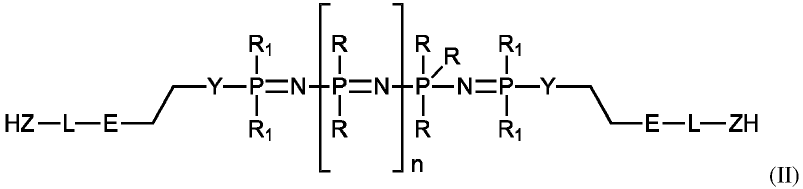 where:
each radical E is, independent of other radicals E, B, Si or S;
each radical L is, independent of other radicals L, an organic radical;
each radical ZH is, independent of other radicals ZH, a group comprising an NCO-reactive hydrogen atom;
each radical R is, independent of other radicals R, an organic radical which does not contain a terminal group with an NCO-reactive hydrogen atom;
each radical R₁ is, independent of other radicals R₁, an organic rest;
each radical Y is, independent of other radicals Y, an organic rest and
n is zero or a positive integer.

8. A polyurethane polymer obtained by reacting a polyphosphazene (I) according to one of claims 1 to 6 with a polyisocyanate.

9. A method of manufacturing a polyurethane polymer comprising reacting a polyphosphazene (I) according to one of claims 1 to 6 with a polyisocyanate.

10. The polymer of claim 8 or the method of claim 9, wherein the polyisocyanate is an NCO-terminated prepolymer obtained by the reaction of a monomeric polyisocyanate and a polyether polyol.

11. The polymer of claim 10 or the method of claim 10, wherein the polyether polyol is a random copolymer of an alkylene oxide and a comonomer selected from: lactides, glycolides, cyclical dicarboxylic acid anhydrides and mixtures of at least two of the aforementioned compounds.

12. A hydrogel obtained by contacting a polyphosphazene (I) according to one of claims 1 to 6 and/or a polyurethane according to claim 8 with water.

13. The hydrogel of claim 12, further comprising, distributed within the hydrogel, a pharmaceutical compound, a pesticide, a herbicide, a pheromone or a combination of at least two of the aforementioned compounds.

14. A hydrogel according to claim 12 for use as a post-operative separator between internal organs of a human or an animal.

15. A hydrogel according to claim 13 for use as a delayed-release formulation for the pharmaceutical compound, pesticide, herbicide, pheromone or combination of at least two of the aforementioned compounds distributed within the hydrogel.

## Patentansprüche

1. Polyphosphazen der allgemeinen Formel (I): 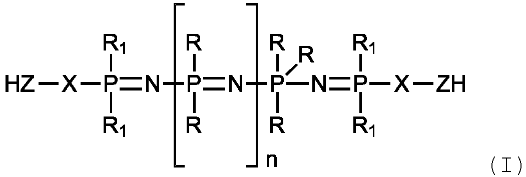 wobei:
jeder Rest R unabhängig von anderen Resten R für einen organischen Rest steht, der keine Endgruppe mit einem NCO-reaktiven Wasserstoffatom enthält;
jeder Rest R₁ unabhängig von anderen Resten R₁ für einen organischen Rest steht;
jeder Rest X unabhängig von anderen Resten X für einen organischen Rest steht;
n für null oder eine positive ganze Zahl steht;
**dadurch gekennzeichnet, dass**
jeder Rest ZH unabhängig von anderen Resten ZH für eine Gruppe mit einem NCO-reaktiven Wasserstoffatom steht.

2. Polyphosphazen nach Anspruch 1, wobei der mindestens eine Rest ZH für eine Hydroxylgruppe, eine primäre Aminogruppe oder eine sekundäre Aminogruppe steht.

3. Polyphosphazen nach Anspruch 1 oder 2, wobei der mindestens eine Rest X eine der folgenden Gruppen umfasst: B-CH₂-CH₂-, -Si-CH₂-CH₂- oder -S-CH₂-CH₂-, wobei sich die -CH₂-CH₂-Gruppen zwischen dem B-, Si- bzw. S-Atom und einem P-Atom, an das Reste R₁ gebunden sind, befinden.

4. Polyphosphazen nach einem der Ansprüche 1 bis 3, wobei mindestens ein Rest X eine der folgenden Gruppen umfasst: -(CH₂)ₘ-B-, -(CH₂)ₘ- Si- oder -(CH₂)ₘ- S-, wobei m für eine ganze Zahl von 1 bis 16 steht, wobei sich die -(CH₂)ₘ-Gruppen zwischen dem B-, Si- bzw. S-Atom und einem Rest ZH befinden.

5. Polyphosphazen nach einem der Ansprüche 1 bis 4, wobei mindestens ein Rest X-ZH die Gruppe -Ar-CH₂-CH₂-S-(CH₂)ₘ-OH oder -Ar-CH₂-CH₂-S-(CH₂)ₘ-NH₂ umfasst, wobei m für eine ganze Zahl von 1 bis 16 steht und Ar für einen Phenylring mit oder ohne zusätzliche Substituenten steht.

6. Polyphosphazen nach einem der Ansprüche 1 bis 5, wobei mindestens ein Rest R ein an ein Phosphoratom einer Phosphazengruppe gebundenes Sauerstoff- oder Stickstoffatom umfasst und/oder mindestens ein Rest R₁ für eine substituierte oder unsubstituierte Phenylgruppe steht.

7. Verfahren zur Herstellung eines Polyphosphazens gemäß der allgemeinen Formel (II), umfassend das Umsetzen einer eine C=C-Doppelbindung tragenden Verbindung der allgemeinen Formel (III) mit einer eine Element-Wasserstoff-Bindung tragenden Verbindung der allgemeinen Formel H-E-L-ZH: 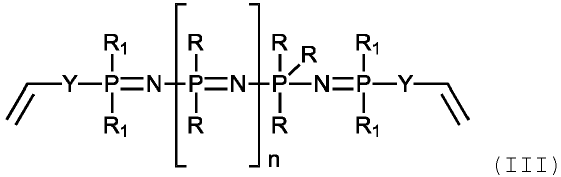 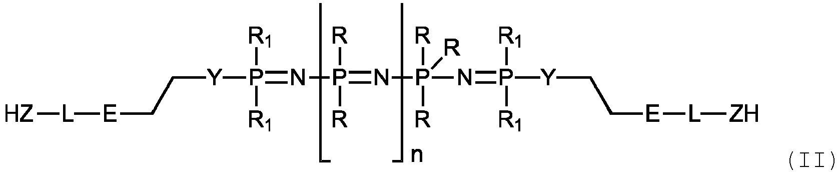 wobei:
jeder Rest E unabhängig von anderen Resten E für B, Si oder S steht;
jeder Rest L unabhängig von anderen Resten L für einen organischen Rest steht;
jeder Rest ZH unabhängig von anderen Resten ZH für eine Gruppe mit einem NCO-reaktiven Wasserstoffatom steht;
jeder Rest R unabhängig von anderen Resten R für einen organischen Rest steht, der keine Endgruppe mit einem NCO-reaktiven Wasserstoffatom enthält;
jeder Rest R₁ unabhängig von anderen Resten R₁ für einen organischen Rest steht;
jeder Rest Y unabhängig von anderen Resten Y für einen organischen Rest steht und
n für null oder eine positive ganze Zahl steht.

8. Polyurethanpolymer, erhalten durch Umsetzen eines Polyphosphazens (I) nach einem der Ansprüche 1 bis 6 mit einem Polyisocyanat.

9. Verfahren zur Herstellung eines Polyurethanpolymers, umfassend das Umsetzen eines Polyphosphazens (I) nach einem der Ansprüche 1 bis 6 mit einem Polyisocyanat.

10. Polymer nach Anspruch 8 oder Verfahren nach Anspruch 9, wobei es sich bei dem Polyisocyanat um ein durch die Umsetzung eines monomeren Polyisocyanats und eines Polyetherpolyols erhaltenes NCO-terminiertes Prepolymer handelt.

11. Polymer nach Anspruch 10 oder Verfahren nach Anspruch 10, wobei es sich bei dem Polyetherpolyol um ein statistisches Copolymer von einem Alkylenoxid und einem aus Lactiden, Glykoliden, cyclischen Dicarbonsäureanhydriden und Mischungen von mindestens zwei der oben aufgeführten Verbindungen ausgewählten Comonomer handelt.

12. Hydrogel, erhalten durch Inkontaktbringen eines Polyphosphazens (I) nach einem der Ansprüche 1 bis 6 und/oder eines Polyurethans nach Anspruch 8 mit Wasser.

13. Hydrogel nach Anspruch 12, ferner umfassend in dem Hydrogel verteilt eine pharmazeutische Verbindung, ein Pestizid, ein Herbizid, ein Pheromon oder eine Kombination von mindestens zwei der oben aufgeführten Verbindungen.

14. Hydrogel nach Anspruch 12 zur Verwendung als postoperativer Separator zwischen inneren Organen eines Menschen oder eines Tiers.

15. Hydrogel nach Anspruch 13 zur Verwendung als eine Formulierung mit verzögerter Freisetzung für die pharmazeutische Verbindung, das Pestizid, das Herbizid, das Pheromon oder die Kombination von mindestens zwei der oben aufgeführten Verbindungen, die bzw. das in dem Hydrogel verteilt ist.

## Revendications

1. Polyphosphazène de la formule générale (I) : 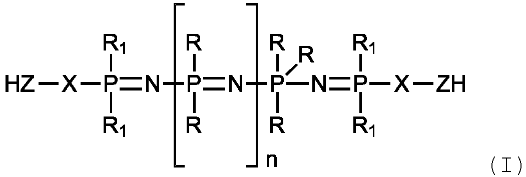 où :
chaque radical R est, indépendamment d'autres radicaux R, un radical organique qui ne contient pas un groupe terminal comportant un atome d'hydrogène réactif avec NCO ;
chaque radical R₁ est, indépendamment d'autres radicaux R₁, un radical organique ;
chaque radical X est, indépendamment d'autres radicaux X, un radical organique ;
n est zéro ou un entier positif ;
**caractérisé en ce que**
chaque radical ZH est, indépendamment d'autres radicaux ZH, un groupe comprenant un atome d'hydrogène réactif avec NCO.

2. Polyphosphazène selon la revendication 1, dans lequel au moins un radical ZH est un groupe hydroxyle, un groupe amino primaire ou un groupe amino secondaire.

3. Polyphosphazène selon la revendication 1 ou 2, dans lequel au moins un radical X comprend l'un des groupes suivants : -B-CH₂-CH₂-, -Si-CH₂-CH₂- ou -S-CH₂-CH₂-, dans lequel les groupes -CH₂-CH₂- sont situés entre les atomes B, Si ou S et un atome P auquel les radicaux R₁ sont liés.

4. Polyphosphazène selon l'une des revendications 1 à 3, dans lequel au moins un radical X comprend l'un des groupes suivants : -(CH₂)ₘ-B-, -(CH₂)ₘ-Si- ou -(CH₂)ₘ-S-, m étant un entier de 1 à 16, dans lequel les groupes - (CH₂)ₘ- sont situés entre les atomes B, Si ou S et un radical ZH.

5. Polyphosphazène selon l'une des revendications 1 à 4, dans lequel au moins un radical X-ZH comprend le groupe -Ar-CH₂-CH₂-S-(CH₂)ₘ-OH ou -Ar-CH₂-CH₂-S-(CH₂)ₘ-NH₂, m étant un entier de 1 à 16 et Ar étant un cycle phényle avec ou sans substituants supplémentaires.

6. Polyphosphazène selon l'une des revendications 1 à 5, dans lequel au moins un radical R comprend un atome d'oxygène ou d'azote lié à un atome de phosphore d'un groupe phosphazène et/ou au moins un radical R₁ est un groupe phényle substitué ou non substitué.

7. Procédé de fabrication d'un polyphosphazène selon la formule générale (II), comprenant la mise en réaction d'un composé portant une double liaison C=C de la formule générale (III) avec un composé portant une liaison élément-hydrogène de la formule générale H-E-L-ZH : 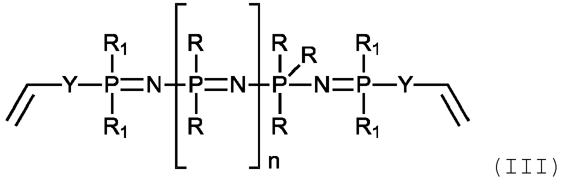 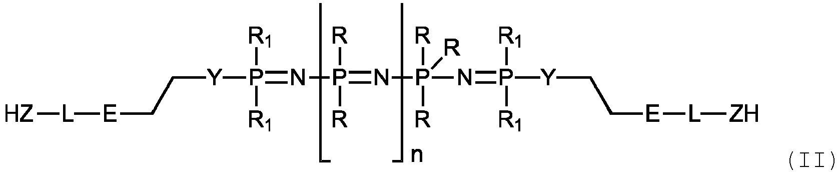 où :
chaque radical E est, indépendamment d'autres radicaux E, B, Si ou S ;
chaque radical L est, indépendamment d'autres radicaux L, un radical organique ;
chaque radical ZH est, indépendamment d'autres radicaux ZH, un groupe comprenant un atome d'hydrogène réactif avec NCO ;
chaque radical R est, indépendamment d'autres radicaux R, un radical organique qui ne contient pas un groupe terminal comportant un atome d'hydrogène réactif avec NCO ;
chaque radical R₁ est, indépendamment d'autres radicaux Rᵢ, un radical organique ;
chaque radical Y est, indépendamment d'autres radicaux Y, un radical organique et
n est zéro ou un entier positif.

8. Polymère de polyuréthane obtenu en mettant en réaction un polyphosphazène (I) selon l'une des revendications 1 à 6 avec un polyisocyanate.

9. Procédé de fabrication d'un polymère de polyuréthane comprenant la mise en réaction d'un polyphosphazène (I) selon l'une des revendications 1 à 6 avec un polyisocyanate.

10. Polymère selon la revendication 8 ou procédé selon la revendication 9, dans lequel le polyisocyanate est un prépolymère à terminaison NCO obtenu par la réaction d'un polyisocyanate monomérique et d'un polyéther polyol.

11. Polymère selon la revendication 10 ou procédé selon la revendication 10, dans lequel le polyéther polyol est un copolymère aléatoire d'un oxyde d'alkylène et d'un comonomère sélectionné parmi : lactides, glycolides, anhydrides d'acides dicarboxyliques cycliques et des mélanges d'au moins deux des composés susmentionnés.

12. Hydrogel obtenu en mettant en contact un polyphosphazène (I) selon l'une des revendications 1 à 6 et/ou un polyuréthane selon la revendication 8 avec de l'eau.

13. Hydrogel selon la revendication 12, comprenant en outre, distribué dans l'hydrogel, un composé pharmaceutique, un pesticide, un herbicide, une phéromone ou une combinaison d'au moins deux des composés susmentionnés.

14. Hydrogel selon la revendication 12 pour une utilisation comme séparateur postopératoire entre des organes internes d'un humain ou d'un animal.

15. Hydrogel selon la revendication 13 pour une utilisation comme formulation à libération retardée pour le composé pharmaceutique, le pesticide, l'herbicide, la phéromone ou une combinaison d'au moins deux des composés susmentionnés distribués dans l'hydrogel.
